# EUROPEAN PATENT SPECIFICATION

(11) **EP 2 222 655 B1**
(45) Date of publication and mention of the grant of the patent: **29.06.2011**
(21) Application number: 08852677.7
(22) Date of filing: 19.11.2008
(51) Int. Cl.: C07D 277/56, A01N 43/78

(54) **N-CYCL0ALKYLMETHYL-2-AMIN0THIAZ0LE-5-CARB0XAMIDES FOR USE IN PLANT DISEASE CONTROL**
N-CYCL0ALKYLMETHYL-2-AMIN0THIAZ0L-5-CARB0XAMIDE ALS PFLANZENSCHUTZMITTEL
COMPOSÉS AMIDES ET LEUR UTILISATION POUR LUTTER CONTRE UNE MALADIE DE PLANTE

(30) Priority: 20.11.2007 JP 2007300139
(43) Date of publication of application: 01.09.2010
(73) Proprietor: Sumitomo Chemical Company, Limited, Tokyo 104-8260 (JP)
(72) Inventor: KOMORI, Takashi, Nerima-ku Tokyo 176-0013 (JP); KURAHASHI, Makoto, Nishinomiya-shi Hyogo 663-8201 (JP)
(74) Representative: Vossius & Partner
(86) International application number: PCT/JP2008/071479
(87) International publication number: WO 2009/066790

(56) References cited:
- US-A- 3 725 427
- MICHAEL HENNINGSEN: "Pilzbekämpfung in der Landwirtschaft Moderne Fungizide" CHEMIE IN UNSERER ZEIT, vol. 37, 9 April 2003 (2003-04-09), pages 98-111, XP002520176

## Description

### Technical Field

The present invention relates to amide compounds and use thereof for plant disease control.

### Background Art

Hitherto, agents for controlling plant diseases have been developed, and compounds having a plant disease controlling effect have been found and put to practical use.

For example US 3 725 427 discloses 2-Aminothiazole-5-carboxamides for use as fungicides. The journal "Chemie in unserer Zeit" 2003,37,98-(1) gives a review on modern fungicides including the aminothiazole Ethaboxam.

### Disclosure of the Invention

### Problems to be Solved by the Invention

An object of the present invention is to provide a compound having excellent plant disease controlling effect.

### Means for Solving the Problems

As a result of intensive research conducted by the present inventors in an attempt to find compounds having excellent plant disease controlling effect, it has been found that amide compounds represented by the following formula (1) have excellent plant disease controlling effect. Thus, the present invention has been accomplished.

That is, the present invention provides an amide compound represented by the formula (1): 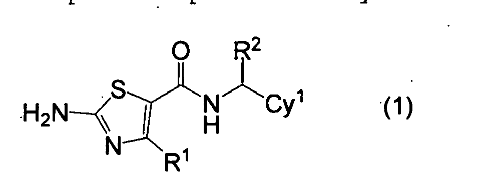 wherein R¹ and R² independently represent a hydrogen atom or a methyl group; and Cy¹ represents a C3-C6 cycloalkyl group) (hereinafter referred to as "the present compound"), a plant disease controlling agent comprising the present compound as an active ingredient (hereinafter, referred to as "the present controlling agent"); and a method for controlling plant diseases including the step of applying an effective amount of the present compound to plants or soils (hereinafter, referred to as "the present controlling method").

### Effect of the Invention

The present compound has excellent plant disease controlling effect, and hence is useful as an active ingredient of plant disease controlling agents.

### Best Mode for Carrying Out the Invention

Examples of the C3-C6 cycloalkyl group represented by Cy¹ include a cyclopropyl group, a cyclobutyl group, a cyclopentyl group and a cyclohexyl group.

Examples of the amide compound represent by the formula (1) include:
an amide compound represented by the formula (1), wherein Cy¹ is a cyclopropyl group;
an amide compound represented by the formula (1), wherein Cy¹ is a cyclobutyl group;
an amide compound represented by the formula (1), wherein Cy¹ is a cyclopentyl group;
an amide compound represented by the formula (1), wherein Cy¹ is a cyclohexyl group;
an amide compound represented by the formula (1), wherein R¹ is a hydrogen atom;
an amide compound represented by the formula (1), wherein R¹ is a methyl group;
an amide compound represented by the formula (1), wherein R² is a hydrogen atom;
an amide compound represented by the formula (1), wherein R² is a methyl group;
an amide compound represented by the formula (1), wherein R² is a methyl group, and Cy¹ is s a cyclopropyl group;
an amide compounds represented by the formula (1), wherein R² is a methyl group, and Cy¹ is a cyclobutyl group;
an amide compound represented by the formula (1), wherein R² is a methyl group, and Cy¹ is a cyclopentyl group; and
an amide compound represented by the formula (1), wherein R² is a methyl group, and Cy¹ is a cyclohexyl group.

Herein, sometimes, two or more isomers are represented by one common structural formula. Such a structural formula includes all of isomers, such as geometric isomers, optical isomers, stereoisomers and tautomers, and isomer mixtures.

Hereinafter, production processes of the present compound will be explained.

The compound (1) can be produced, for example, by the following Process 1 to Process 3.

### Process 1

The compound (1) or a salt thereof (e.g., hydrochloride) can be produced by reacting the compound (3) or a salt thereof (e.g., hydrochloride and hydrobromide) with the compound (4) in the presence of a dehydration condensation agent. 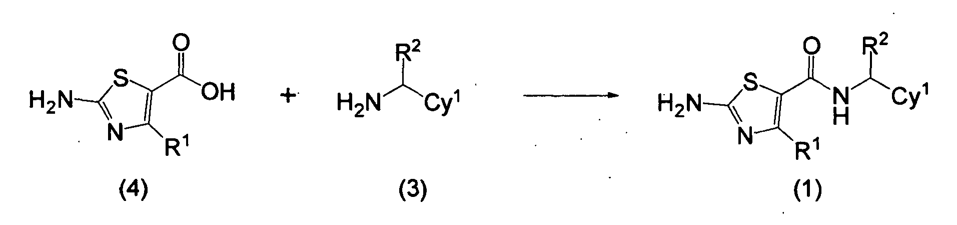 wherein R¹ and R² independently represent a hydrogen atom or a methyl group; and Cy¹ represents a C3-C6 cycloalkyl group.

This reaction is usually carried out in the presence of a solvent.

Examples of the solvent for the reaction include ethers such as tetrahydrofuran (hereinafter sometimes referred to as "THF"), ethylene glycol dimethyl ether and tert-butylmethyl ether (hereinafter sometimes referred to as "MTBE"); aliphatic hydrocarbons such as hexane, heptane and octane; aromatic hydrocarbons such as toluene and xylene; halogenated hydrocarbons such as chlorobenzene; esters such as butyl acetate and ethyl acetate; nitriles such as acetonitrile; acid amides such as N,N-dimethylformamide (hereinafter sometimes referred to as "DMF"); sulfoxides such as dimethyl sulfoxide (hereinafter sometimes referred to as "DMSO"); and mixtures thereof.

Examples of the dehydration condensation agent for the reaction include such as 1-ethyl-3-(3-dimethylaminopropyl)carbodiimide hydrochloride (hereinafter referred to as "WSC"), (benzotriazol-1-yloxy)tris(dimethylamino)phosphonium hexafluorophosphate (hereinafter referred to as "BOP reagent") and 1,3-dicyclohexylcarbodiimide.

The compound (3) is usually used in a proportion of 1 to 3 moles, and the dehydration condensation agent is usually used in a proportion of 1 to 5 moles per mole of the compound (4).

The reaction temperature is usually in a range from 0 to 200°C, and the reaction time is usually in a range from 1 to 24 hours.

When the BOP reagent is used in the reaction, the reaction can be carried out in the presence of a base, if necessary. Examples of the base include tertiary amines such as triethylamine and diisopropylethylamine; and nitrogen-containing aromatic compounds such as pyridine and 4-dimethylaminopyridine.

The base is usually used in a proportion of 1 to 10 moles per mole of the compound (4).

After completion of the reaction, the compound (1) can be isolated by subjecting the reaction mixture to post-treatment such as adding water to the reaction mixture, followed by extraction with an organic solvent, drying and concentrating the organic layer or the like. The present compound (1) thus isolated can be purified further by subjecting it to chromatography, recrystallization or the like.

### Process 2

The compound (1) can be produced by reacting the compound (3) or a salt thereof (e.g., hydrochloride and hydrobromide) with the compound (5) or a salt thereof (e.g., hydrochloride) in the presence of a base: 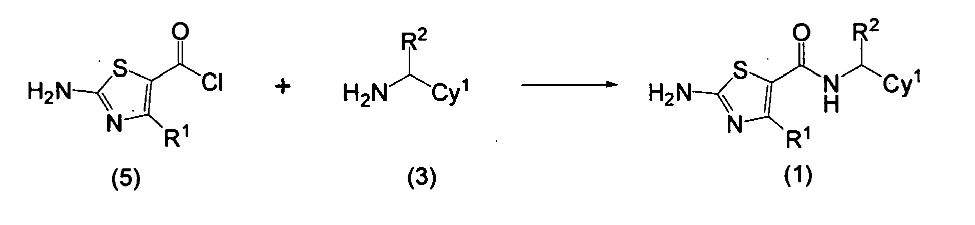 wherein R¹, R² and Cy¹ are as defined above.

This reaction is usually carried out in the presence of a solvent.

Examples of the solvent for the reaction include ethers such as THF, ethylene glycol dimethyl ether and MTBE; aliphatic hydrocarbons such as hexane, heptane and octane; aromatic hydrocarbons such as toluene and xylene; halogenated hydrocarbons such as chlorobenzene; esters such as butyl acetate and ethyl acetate; nitriles such as acetonitrile; acid amides such as DMF; sulfoxides such as DMSO; and mixtures thereof.

Examples of the base for the reaction include alkali metal carbonates such as sodium carbonate and potassium carbonate; tertiary amines such as triethylamine and diisopropylethylamine; and nitrogen-containing aromatic compounds such as pyridine and 4-dimethylaminopyridine.

The compound (3) is usually used in a proportion of 1 to 3 moles, and the base is usually used in a proportion of 1 to 10 moles per mole of the compound (5).

The reaction temperature is usually in a range from - 20 to 140°C, and the reaction time is usually in a range from 0.1 to 24 hours.

After completion of the reaction, the present compound (1) can be isolated by subjecting the reaction mixture to post-treatment such as extracting the reaction mixture with an organic solvent, followed by drying and concentrating the organic layer. The present compound (1) thus isolated can be purified further by subjecting it to chromatography, recrystallization or the like.

### Process 3

The compound (1) can be produced from the compound (6), for example, in accordance with the following scheme. 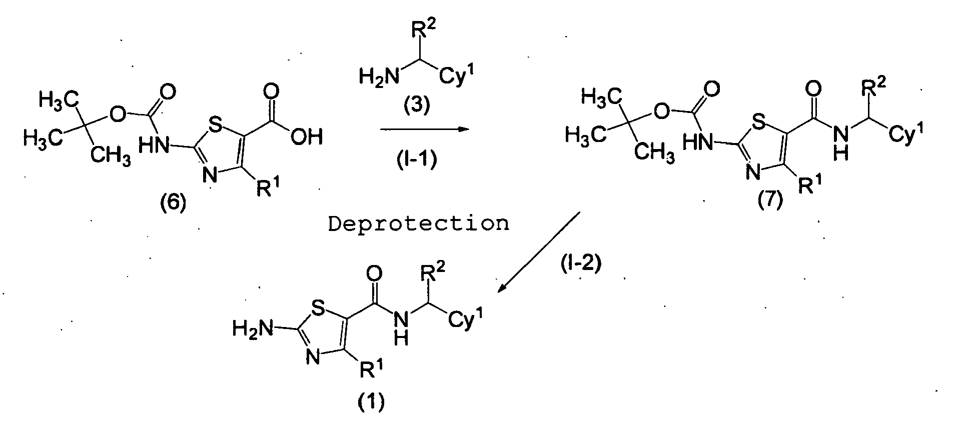 wherein R¹, R² and Cy¹ are as defined above.

### Step (I-1)

The compound (7) can be produced by reacting the compound (6) with the compound (3) or a salt thereof (e.g., hydrochloride and hydrobromide) in the presence of a dehydration condensation agent.

This reaction is usually carried out in the presence of a solvent.

Examples of the solvent for the reaction include ethers such as THF, ethylene glycol dimethyl ether and MTBE; aliphatic hydrocarbons such as hexane, heptane and octane; aromatic hydrocarbons such as toluene and xylene; halogenated hydrocarbons such as chlorobenzene; esters such as butyl acetate and ethyl acetate; nitriles such as acetonitrile; acid amides such as DMF; sulfoxides such as DMSO; and mixtures thereof.

Examples of the dehydration condensation agent for the reaction include such as WSC, BOP reagent and 1,3-dicyclohexylcarbodiimide.

The compound (3) is usually used in a proportion of 1 to 3 moles, and the dehydration condensation agent is usually used in a proportion of 1 to 5 moles per mole of the compound (6).

The reaction temperature is usually in a range from 0 to 200°C, and the reaction time is usually in a range from 1 to 24 hours.

When the BOP reagent is used in the reaction, the reaction can be carried out in the presence of a base, if necessary. Examples' of the base include tertiary amines such as triethylamine and diisopropylethylamine; and nitrogen-containing aromatic compounds such as pyridine and 4-dimethylaminopyridine.

The base is usually used in a proportion of 1 to 10 moles per mole of the compound (6).

After completion of the reaction, the compound (7) can be isolated by subjecting the reaction mixture to post-treatment such as adding water to the reaction mixture, followed by extraction with an organic solvent, drying and concentrating the organic layer. The compound (7) thus isolated'can be purified further by subjecting it to chromatography, recrystallization or the like.

### Step (I-2)

The compound (1) can be produced by deprotection of the 1,1-dimethylethylcarbamate group in the compound (7).

For example, when the deprotection is carried out using an acid, the reaction is usually carried out in the presence of a solvent.

Examples of the solvent include aromatic hydrocarbons such as toluene and xylene; halogenated hydrocarbons such as methylene chloride, chloroform and chlorobenzene; sulfoxides such as DMSO; alcohols such as methanol, ethanol and 2-methylethanol; ketones such as acetone, methyl ethyl ketone and methyl isobutyl ketone; water; and mixtures thereof.

Examples of the acid for the reaction include inorganic acids such as hydrochloric acid and sulfuric acid; and organic acids such as trifluoroacetic acid, p-toluenesulfonic acid and methanesulfonic acid.

The acid is usually used in a proportion of 1 mole to excess amount per mole of the compound (7).

The reaction temperature is usually in a range from 0 to 150°C, and the reaction time is usually in a range from 0.1 to 24 hours.

After completion of the reaction, the compound (1) can be isolated by subjecting the reaction mixture to post-treatment such as extracting the reaction mixture with an organic solvent, followed by drying and concentrating the organic layer. The present compound (1) thus isolated can be purified further by subjecting it to chromatography, recrystallization or the like.

Some of the intermediates used for the production of the present compound are compounds commercially available or disclosed in known literatures or the like.

While the present controlling composition can be composed only of the present compound, it is usually used in a formulation form such as wettable powders, water dispersible granules, flowable concentrates, granules, dry flowable concentrates, emulsifiable concentrates, aqueous liquid formulations, oil formulations, smoking formulations, aerosols, microcapsules or the like, by mixing the present compound with a carrier (e.g., a solid, liquid or gaseous carrier) and auxiliary agents for formulation such as surfactants, binders, dispersants and stabilizers. Such formulations usually contain the present compound in an amount of 0.1 to 99% by weight, preferably 0.2 to 90% by weight.

Examples of the solid carrier used for the formulation procedure include fine powders or particles of clays (e.g., kaolin, diatomaceous earth, synthetic hydrous silicon oxide agalmatolite clay, bentonite, acid clay and talc) and other inorganic minerals (e.g., sericite, quartz powder, sulfur powder, activated carbon, calcium carbonate and hydrated silica); and examples of the liquid carrier include such as water, alcohols (e.g., methanol and ethanol), ketones (e.g., acetone and methyl ethyl ketone), aromatic hydrocarbons (e.g., benzene, toluene, xylene, ethylbenzene and methylnaphthalene), aliphatic or alicyclic hydrocarbons (e.g., n-hexane, cyclohexanone and kerosene), esters (e.g., ethyl acetate and butyl acetate), nitriles (e.g., acetonitrile and isobutyronitrile), ethers (e.g., dioxane and diisopropyl ether), acid amides (e.g., dimethylformamide and dimethylacetoamide) and halogenated hydrocarbons (e.g., dichloroethane, trichloroethylene and carbon tetrachloride).

Examples of the surfactant include such as alkyl sulfates, alkylsulfonates, alkylarylsulfonates, alkylaryl ethers and polyoxyethylenated products thereof, polyoxyethylene glycol ethers, polyhydric alcohol esters and sugar alcohol derivatives.

Examples of the other auxiliary agents for formulation include such as binders, dispersants, thickening agents, wetting agents, extending agents and antioxidants. Specific example thereof include such as casein, gelatin, polysaccharides (e.g., starch, gum arabic, cellulose derivatives and alginic acid), lignin derivatives, bentonite, saccharides, synthetic water-soluble polymers (e.g., polyvinyl alcohols, polyvinylpyrrolidones and polyacrylic acids), PAP (acidic isopropyl phosphate), BHT (2,6-di-tert-butyl-4-methylphenol), BHA (a mixture of 2-tert-butyl-4-methoxyphenol and 3-tert-butyl-4-methoxyphenol), vegetable oils, mineral oils, and fatty acids and esters thereof.

Although there is no particular limitation on a method for applying the present controlling agent in order to control plant diseases, the method is exemplified by treatment of plants such as foliar application, treatment of planting sites such as soil treatment, and treatment of seeds such as seed disinfection.

The present controlling agent can also be used in a mixture form with other fungicides, insecticides, acaricides, nematicides, herbicides, plant growth regulators, safeners, fertilizers or soil conditioners. It is also possible to use the present controlling agent simultaneously with such other chemicals without mixing with them.

Examples of the fungicides used with the present controlling agent include as follows.
(1) Azole fungicides:
   propiconazole, prothioconazole, triadimenol, prochloraz, penconazole, tebuconazole, flusilazole, diniconazole, bromuconazole, epoxiconazole, difenoconazole cyproconazole, metconazole, triflumizole, tetraconazole, microbutanil, fenbuconazole, hexaconazole, fluquinconazole, triticonazole, bitertanol, imazalil, flutriafol, simeconazole, ipconazole, and the like;
(2) Amine fungicides:
   fenpropimorph, tridemorph, fenpropidin, spiroxamine, and the like;
(3) Benzimidazole fungicides:
   carbendazim, benomyl, thiabendazole, thiophanate-methyl, and the like;
(4) Dicarboxyimide fungicides:
   procymidone, iprodione, vinclozolin, and the like;
(5) Anilino pyrimidine fungicides:
   cyprodinil, pyrimethanil, mepanipyrim, and the like;
(6) Phenylpyrrole fungicides:
   fenpiclonil, fludioxonil, and the like;
(7) Strobilurin fungicides:
   kresoxim-methyl, azoxystrobin, trifloxystrobin, fluoxastrobin, picoxystrobin, pyraclostrobin, dimoxystrobin, pyribencarb, metominostrobin, oryzastrobin, enestrobin, and the like;
(8) Phenyl amide fungicides:
   metalaxyl, metalaxyl-M or mefenoxam, benalaxyl, benalaxyl-M or kiralaxyl, and the like;
(9) Carboxylic acid amide fungicides:
   dimethomorph, iprovalicarb, benthiavalicarb-isopropyl, mandipropamid, valiphenal;
(10) Carboxamide fungicides:
   carboxin, mepronil, flutolanil, thifluzamide, furametpyr, boscalid, penthiopyrad, fluopyram, bixafen;
(11) Other fungicides:
   diethofencarb; thiram; fluazinam; mancozeb; chlorothalonil; captan; dichlofluanide; folpet; quinoxyfen; fenhexamide; famoxadone; fenamidone; zoxamide; ethaboxam; amisulbrom; cyazofamid; metrafenone; cyflufenamid; proquinazid; flusulfamide; fluopicolide; fosetyl; cymoxanil; pencycuron; tolclofos-methyl; carpropamide; diclocymet; fenoxanil; tricyclazole; pyroquilon; probenazole; isotianil; tiadinil; tebufloquine; diclomezine; kasugamycin; ferimzone; fthalide; validamycin; hydroxyisoxazole; iminoctadine acetate; isoprothiolane; oxolinic acid; oxytetracycline; streptomycin; basic copper chloride; copper (II) hydroxide; basic copper sulfate; organic copper; sulfur, and the like; a pyrazole carboxamide compound represented by the formula (A) : 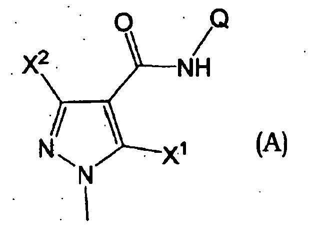 wherein X¹ represents a hydrogen atom or a halogen atom; X² represents a methyl group, a difluoromethyl group or a trifluoromethyl group; and Q represents any of the following groups; 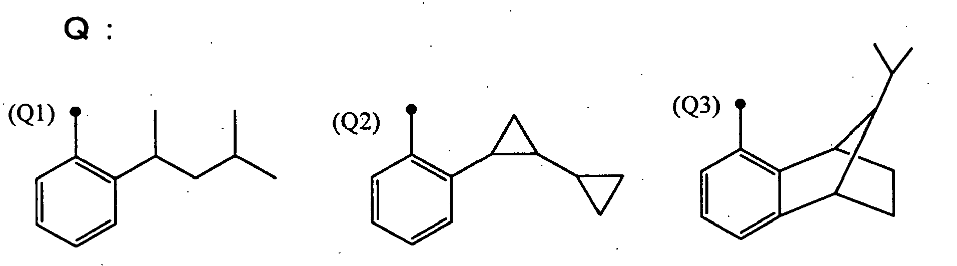 an α-alkoxyphenylacetic acid compound represented by the formula (B): 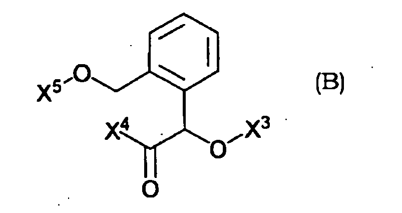
   wherein X³ represents a methyl group, a difluoromethyl group or an ethyl group; X⁴ represents a methoxy group or a methylamino group; and X⁵ represents a phenyl group, a 2-methylphenyl group or a 2,5-dimethylphenyl group;
      and a pyrazolinone compound represented by the formula (C): 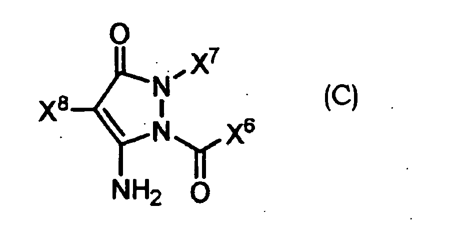
   wherein X⁶ represents a methoxy group, an ethoxy group, a propoxy group, a 2-propenyloxy group, a 2-propinyloxy group, a 3-butenyloxy group, a 3-butynyloxy group, a methylthio group, an ethylthio group or a 2-propenylthio group; X⁷ represents a 1-methylethyl group or a 1-methylpropyl group; and X⁸ represents a 2-methylphenyl group or a 2,6-dichlorophenyl group.

Examples of the insecticides used with the present controlling agent include as follows.
(1) Organic phosphorus compounds:
   acephate, Aluminium phosphide, butathiofos, cadusafos, chlorethoxyfos, chlorfenvinphos, chlorpyrifos, chlorpyrifos-methyl, cyanophos:CYAP, diazinon, DCIP(dichlorodiisopropyl ether), dichlofenthion:ECP, dichlorvos:DDVP, dimethoate, dimethylvinphos, disulfoton, EPN, ethion, ethoprophos, etrimfos, fenthion:MPP, fenitrothion:MEP, fosthiazate, formothion, Hydrogen phosphide, isofenphos, isoxathion, malathion, mesulfenfos, methidathion:DMTP, monocrotophos, naled:BRP, oxydeprofos:ESP, parathion, phosalone, phosmet:PMP, pirimiphos-methyl, pyridafenthion, quinalphos, phenthoate:PAP, profenofos, propaphos, prothiofos, pyraclorfos, salithion, sulprofos, tebupirimfos, temephos, tetrachlorvinphos, terbufos, thiometon, trichlorphon:DEP, vamidothion, phorate, cadusafos, and the like;

(2) Carbamate compounds:
   alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb:MIPC, metolcarb, methomyl, methiocarb, NAC, oxamyl, pirimicarb, propoxur:PHC, XMC, thiodicarb, xylylcarb, aldicarb, and the like;
(3) Synthetic pyrethroid compounds:
   acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin), furamethrin, tau-fluvalinate, 2,3,5,6-tetrafluoro-4-(methoxymethyl)benzyl(EZ)-(1RS, 3RS;1RS, 3SR)-2,2-dimethyl-3-prop-1-enylcyclopropanecarboxylate, 2,3,5,6-tetrafluoro-4-methylbenzyl(EZ)-(1RS, 3RS:1RS, 3SR)-2,2-dimethyl-3-prop-1-enylcyclopropanecarboxylate, 2,3,5,6-tetrafluoro-4-(methoxymethyl)benzyl(1RS, 3RS;1RS, 3SR)-2,2-dimethyl-3-(2-methyl-1-propenyl)cyclopropanecarboxylate, and the like;
(4) Nereistoxin compounds:
   cartap, bensultap, thiocyclam, monosultap, bisultap, and the like;
(5) Neonicotirioid compounds:
   imidacloprid, nitenpyram, acetamiprid, thiamethoxam, thiacloprid, dinotefuran, clothianidin, and the like;
(6) Benzoyl urea compounds:
   chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, triazuron, and the like;
(7) Phenylpyrazole-based compounds:
   acetoprole, ethiprole, fipronil, vaniliprole, pyriprole, pyrafluprole, and the like;
(8) Bt toxin insecticides:
   Living spores, produced crystalline toxins and the mixtures thereof derived form Baccilus thuringiensis;
(9) Hydrazine compounds:
   chromafenozide, halofenozide, methoxyfenozide, tebufenozide, and the like;
(10) Organic chlorine compounds:
   aldrin, dieldrin, dienochlor, endosulfan, methoxychlor, and the like;
(11) Natural insecticides:
   machine oil and nicotine-sulfate;
(12) Other insecticides:
   avermectin-B, bromopropylate, buprofezin, chlorphenapyr, cyromazine, D-D(1,3-Dichloropropene, emamectin-benzoate, fenazaquin, flupyrazofos, hydroprene, methoprene, indoxacarb, metoxadiazone, milbemycin-A, pymetrozine, pyridalyl, pyriproxyfen, spinosad, sulfluramid, tolfenpyrad, triazamate, flubendiamide, lepimectin, Arsenic acid, benclothiaz, Calcium cyanamide, Calcium polysulfide, chlordane, DDT, DSP, flufenerim, flonicamid, flurimfen, formetanate, metam-ammonium, metam-sodium, Methyl bromide, nidinotefuran, Potassium oleate, protrifenbute, spiromesifen, Sulfur, metaflumizone, spirotetramat, pyrifluquinazone, spinetoram, chlorantraniliprole),

A compound represented by the following formula (D): 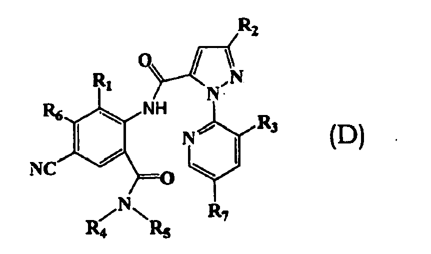 wherein R₁ represents Me, Cl, Br or F;
R₂ represents F, Cl, Br, C1-C4 haloalkyl or C1-C4 haloalkoxy;
R₃ represents F, Cl or Br;
R₄ represents H, one or more halogen atoms, CN, SMe, S(O)Me, or C1-C4 alkyl, C3-C4 alkenyl, C3-C4 alkynyl or C3-C5 cycloalkylalkyl optionally substituted by S(O)₂Me and OMe; R₅ represents H or Me;
R₆ represents H, F or Cl; and
R₇ represents H, F or Cl;
and

A compound represented by the following formula (E): 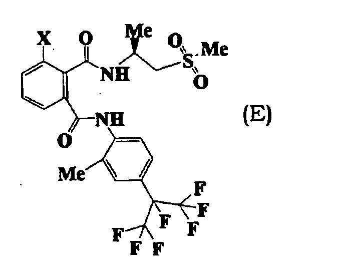 wherein X represents Cl, Br or I.

Examples of the acaricides (acaricidal active ingredients) used with the present controlling agent include such as acequinocyl, amitraz, benzoximate, bifenazate, bromopropylate, chinomethionate, chlorobenzilate, CPCBS(chlorfenson), clofentezine, cyflumetofen, dicofol, etoxazole, fenbutatin oxide, fenothiocarb, fenpyroximate, fluacrypyrim, fluproxyfen, hexythiazox, propargite:BPPS, polynactins, pyridaben, Pyrimidifen, tebufenpyrad, tetradifon, spirodiclofen, spiromesifen, spirotetramat, amidoflumet and cyenopyrafen.

Examples of the nematocides (nematocidal active ingredients) used with the present controlling agent include such as DCIP, fosthiazate, levamisol, methyisothiocyanate, morantel tartarate and imicyafos.

Examples of the safener (safener active ingredients) used with the present controlling agent include such as 1,8-naphthalic anhydride, cyometrinil, oxabetrinil, fluxofenim, flurazole, benoxacor, dichlormid, furilazole, fenclorim, daimuron, cumyluron, dimepiperate, cloquintocetmexyl, fenchlorazole-ethyl, mefenpyr-diethyl and isoxadifen-ethyl.

Examples of the plant growth regulators (plant growth regulating active ingredients) used with the present controlling agent include such as ethephon, chlormequatchloride, mepiquat-chloride, and the like.

It is also possible to obtain high "crop growth improvement effect" in an efficient and labor-saving manner by applying the present controlling agent to a crop imparted with herbicidal resistance in some way, and applying a certain kind of herbicide at the same or different time. Herein, the "crop growth improvement effect" denotes an effect bringing about an increase in crop yield, as a result of controlling damages of crops by insects, diseases and weeds, or the like.

Specifically, the present controlling agent and an imidazolinone herbicide such as imazapyr are applied to a crop imparted with resistance to imidazolinone-based herbicides, such as Clearfield^{®} canola, at the same or different time, so as to improve the growth of Clearfield^{®} canola. Further, the present controlling agent and glyphosate are applied to a crop imparted with resistance to glyphosate, such as RoundupReady^{®} cotton and RoundupReady 2^{®} soybean, at the same or different time, so as to improve the growth of RoundupReady corn and RoundupReady 2 soybean. Further, the present controlling agent and glufosinate are applied to a crop imparted with resistance to glufosinate, such as LibertyLink^{®} corn, at the same or different time, so as to improve the growth of LibertyLink cotton.

Although the applying dosage of the present controlling agent is varied depending on weather conditions, formulation forms, when, how and where the present controlling agent is applied, target diseases, target crops and the like, it is usually 1 to 500 g, preferably 2 to 200g, per 10 are in terms of the present compound in the present controlling agent. When the present controlling agent takes a form of emulsifiers, wettable powders, suspensions or the like, it is usually applied after diluted with water. In this case, the concentration of the present compound after dilution is usually 0.0005 to 2% by weight, preferably 0.005 to 1% by weight. When the present controlling agent takes a form of powders, granules or the like, it is applied as it is without dilution. In an application to seeds, the applying dosage is usually in a range from 0.001 to 100 g, preferably 0.01 to 50 g, per kilogram of seed in terms of the present compound in the present controlling agent.

The present controlling agent can be used as a controlling composition for plant diseases in crop lands such as upland field, paddy field, lawn and turf, orchard and the like. The present controlling agent is able to control plant diseases in the crop lands or the like where the following "crops" and the like are cultivated.

Field crops: corn, rice, wheat, barley, rye, oat, sorghum, cotton, soybean, peanut, buckwheat, sugar beet, rape, sunflower, sugarcane, tobacco, etc.

Vegetables: solanaceae (e.g. eggplant, tomato, green pepper, chili pepper and potato), Cucurbitaceae (e.g. cucumber, pumpkin, zucchini, watermelon and melon), Cruciferae (e.g. Japanese radish, turnip, horseradish, kohlrabi, Chinese cabbage, cabbage, leaf mustard, broccoli and cauliflower), Compositae (e.g. edible burdock, garland chrysanthemum, globe artichoke and lettuce), Liliacede (e.g., Welsh onion, onion, garlic and asparagus), Umbelliferae (e.g. carrot, parsley, celery and parsnip), Chenopodiaceae (e.g. spinach and chard), Lamiaceae (e.g. perilla, mint and basil), strawberry, sweet potato, Chinese yam, taro, jatropha, etc.

Flowers and ornament plants.

Ornamental foliage plants.

Fruit trees: pomaceous fruits (e.g. apple, pear, Japanese pear, Chinese quince and quince), stone fruits (e.g. peach, plum, nectarine, Japanese apricot, yellow peach, apricot and prune), citrus fruits (e.g. satsuma mandarin, orange, lemon, lime and grapefruit), nut trees (e.g. chestnut, walnut, hazel, almond, pistachio, cashew nut and macadamia nut), berries (blueberry, cranberry, blackberry and raspberry), grape, Japanese persimmon, olive, loquat, banana, coffee, date palm, coconut, etc.

Trees other than fruit trees: tea, mulberry, flowering trees and shrubs, street trees (e.g. Japanese ash, birch, flowering dogwood, blue gum, ginkgo, lilac, maple, oak, poplar, Chinese redbud, Formosa sweet gum, plane tree, zelkova, Japanese arborvitae, fir, Japanese hemlock, needle juniper, pine, Japanese spruce and Japanese yew), etc.

The above-mentioned "crops" also include those imparted with resistance to herbicides, such as HPPD inhibitors (e.g., isoxaflutole), ALS inhibitors (e.g., imazethapyr and thifensulfuron-methyl), EPSP synthetase inhibitors, glutamine synthetase inhibitors, bromoxynil and dicamba, by way of a classic breeding method or genetic recombination technology.

Examples of the "crops" imparted with resistance by the classic breeding method include Clearfield^{®} canola resistant to imidazolinone-based herbicides (e.g., imazethapyr), STS soybean resistant to sulfonylurea-based ALS inhibition type herbicides such as thifensulfuron-methyl, or the like. Further, examples of the crops imparted with resistance by the genetic recombination technology include corn cultivars resistant to glyphosate and gluphosinate, which have been already on the market under the trade name of RoundupReady^{®}, RoundupReady 2^{®} and LibertyLink^{®}.

The above-mentioned "crops" also include plants in which the genetic recombination technology has enabled to synthesize, for example, a selective toxin known as genus Bacillus.

Examples of toxins produced in such genetically modified plants include insecticidal proteins derived from Bacillus cereus and Bacillus popilliae; insecticidal proteins such as δ-endotoxins (e.g. Cry1Ab, Cry1Ac, Cry1F, Cry1Fa2, Cry2Ab, Cry3A, Cry3Bb1 and Cry9C), VIP1, VIP2, VIP3 and VIP3A, which are derived from Bacillus thuringiensis; toxins derived from nematodes; toxins produced by animals, such as scorpion toxin, spider toxin, bee toxin and insect-specific neurotoxins; filamentous fungi toxins; plant lectins; agglutinin; protease inhibitors such as trypsin inhibitors, serine protease inhibitor, patatin, cystatin and_papain inhibitors; ribosome-inactivating proteins (RIP) such as ricin, corn-RIP, abrin, rufin, sapolin and priodin; steroid metabolic enzymes such as 3-hydroxysteroid oxidase, ecdysteroid-UDP-glucosyltransferase and cholesterol oxidase; ecdysone inhibitors; HMG-COA reductase; ion channel inhibitors such as a sodium channel inhibitors and calcium channel inhibitors; juvenile hormone esterase; diuretic hormone acceptors; stilbene synthetase; bibenzyl synthetase; chitinase; and glucanase.

The toxins produced in such genetically modified crops also include hybrid toxins, partially deficient toxins and modified toxins of insecticidal proteins, such as δ-endotoxin proteins (e.g. Cry1Ab, Cry1Ac, Cry1F Cry1Fa2, Cry2Ab, Cry3A, Cry3Bb1 and Cry9C), VIP1, VIP2, VIP3 and VIP3A. The hybrid toxins are produced by a novel combination of the different domains of such a protein by adopting recombination technology. The known partially deficient toxin is Cry1Ab, in which a part of amino acid sequence is deficient. In the modified toxins, one or a plurality of amino acids of a natural toxin are replaced.

Examples of such toxins and genetically modified plants capable of synthesizing such toxins are described in EP-A-0 374 753, WO 93/07278, WO 95/34656, EP-A-0 427 529, EP-A-451 878, WO 03/052073, etc.

The toxins contained in such genetically modified plants impart resistance to insect pests of Coleoptera, insect pests of Diptera and insect pests of Lepidoptera to the plants.

Further, it has already been known that there are genetically modified plants containing one or a plurality of insecticidal pest-resistant genes and capable of producing one or a plurality of toxins. Some of them are commercially available. Examples of such genetically modified plants include such as YieldGard^{®} (a corn cultivar capable of producing a Cry1Ab toxin), YieldGard Rootworm^{®} (a corn cultivar capable of producing a Cry3Bb1 toxin), YieldGard Plus^{®} (a corn cultivar capable of producing Cry1Ab and Cry3Bb1 toxins), Herculex I^{®} (a corn cultivar capable of producing phosphinotrysin N-acetyltransferase (PAT) for imparting resistance to a Cry1Fa2 toxin and Glufosinate), NuCOTN33B (a cotton cultivar capable of producing a Cry1Ac toxin), Bollgard I^{®} (a cotton cultivar capable of producing a Cry1Ac toxin), Bollgard II^{®} (a cotton cultivar capable of producing Cry1Ab and Cry2Ab toxins), VIPCOT^{®} (a cotton cultivar capable of producing a VIP toxin), NewLeaf^{®} (a potato cultivar capable of producing a Cry3A toxin), NatureGard^{®} Agrisure^{®} GT Advantage (GA21 Glyphosate resistant trait), Agrisure^{®} CB Advantage (Bt11 corn borer (CB) trait), and Protecta^{®}.

The above-mentioned "crops" also include those imparted with an ability of producing an anti-pathogenic substance having selective action, by way of genetic recombination technology.

As examples of the anti-pathogenic substance, PR proteins and the like are known (PRPs, EP-A-0 392 225). Such anti-pathogenic substances and genetically modified plants capable of producing them are described in EP-A-0 392 225, WO 95/33818, EP-A-0 353 191, etc.

Examples of such anti-pathogenic substances produced by the genetically modified plants include ion channel inhibitors, such as sodium channel inhibitors and calcium channel inhibitors (for example, KP1, KP4 and KP6 toxins produced by viruses are known); stilbene synthases; bibenzyl synthases; chitinase; glucanase; PR proteins; and anti-pathogenic substances produced by microorganisms, such as peptide antibiotics, antibiotics having a heterocyclic ring and protein factors involved in plant disease resistance (which are called as plant-disease-resistant genes and are described in WO 03/000906), etc.

The above-mentioned "crops" also include the following strains: a strain imparted with two or more traits relating to the above-mentioned herbicide resistance, pest resistance, disease resistance and the like, by means of classic breeding technique or genetic recombination technology; and a strain imparted with two or more properties descended from the parents strains, by means of crossbreed between genetically modified plants with similar or different properties.

Examples of plant diseases controllable by the present invention include such as fungal diseases. More specifically, the following plant diseases are listed, but the diseases are not limited thereto.

The present controlling method is usually practiced in the method, wherein the present controlling agent is applied in the above-mentioned manner.

Blast *(Magnaporthe grisea),* Brown spot *(Cochliobolus miyabeanus),* sheath blight *(Rhizoctonia solani)* and "Bakanae" disease *(Gibberella fujikuroi)* of rice; Disease of wheat: powdery mildew *(Erysiphe graminis),* scab *(Fusarium graminearum, F. avenacerum, F. culmorum, Microdochium nivale),* rust *(Puccinia striiformis, P. graminis, P. recondita),* Snow mold *(Micronectriella nivale), Typhula* snow blight (*Typhula* sp.), loose smut *(Ustilago tritici),* bunt *(Tilletia caries),* eyespot *(Pseudocercosporella herpotrichoides),* leaf blotch *(Mycosphaerella graminicola),* glume blotch *(Stagonospora nodorum)* and tan spot *(Pyrenophora tritici-repentis);* Disease of barley: powdery mildew *(Erysiphe graminis),* scab *(Fusarium graminearum, F. avenacerum, F. culmorum, Microdochium nivale),* rust *(Puccinia striiformis, P. graminis, P. hordei),* loose smut *(Ustilago nuda),* scald *(Rhynchosporium secalis),* net blotch *(Pyrenophora teres),* spot blotch *(Cochliobolus sativus),* leaf stripe *(Pyrenophora graminea)* and seedling damping-off by *Rhizoctonia* genus *(Rhizoctonia solani);*
melanose *(Diaporthe citri),* scab *(Elsinoe fawcetti)* and *Penicillium* rot *(Penicillium digitatum, P. italicum)* of citrus;
blossom blight *(Monilinia mali),* canker *(Valsa ceratosperma),* powdery mildew *(Podosphaera leucotricha), Alternaria* leaf spot *(Alternaria alternata* apple pathotype), scab *(Venturia inaequalis)* and anthracnose *(Glomerella cingulata)* of apple;
scab *(Venturia nashicola, V. pirina),* black spot *(Alternaria alternata* Japanese pear pathotype) and rust *(Gymnosporangium haraeanum)* of pear;
brown rot *(Monilinia fructicola),* scab *(Cladosporium carpophilum)* and *Phomopsis* rot (*Phomopsis* sp.) of peach; anthracnose *(Elsinoe ampelina),* ripe rot *(Glomerella cingulata),* powdery mildew *(Uncinula necator)*, rust (*Phakopsora ampelopsidis*)*,* black rot (*Guignardia bidwellii*) and downy mildew *(Plasmopara viticola)* of grape; anthracnose (*Gloeosporium* kaki) and leaf spot (*Cercospora* kaki, *Mycosphaerella nawae*) of Japanese persimmon; anthracnose (Colletotrichum lagenarium), powdery mildew (*Sphaerotheca fuliginea*)*,* gummy stem blight (*Mycosphaerella melonis*)*,* stem rot *(Fusarium oxysporum),* downy mildew *(Pseudoperonospora cubensis), Phytophthora* rot (*Phytophthora* sp.) and seedling blight (*Pythium* sp.) of melons and cucumber;
early blight (*Alternaria solani*)*,* leaf mold (*Cladosporium fulvum*) and leaf blight (*Phytophthora infestans*) of tomato; brown spot (*Phomopsis vexans*) and powdery mildew *(Erysiphe cichoracearum)* of eggplant;
*Alternaria* leaf spot *(Alternaria japonica)* and white spot *(Cercosporella brassicae)* of vegetables of Crusiferae; Welsh onion rust *(Puccinia allii);*
purple stain *(Cercospora kikuchii), Sphaceloma* scab *(Elsinoe glycines),* pod and stem blight (*Diaporthe phaseolorum* var. sojae) and rust *(Phakopsora pachyrhizi)* of soybean;
kidney bean anthracnose *(Colletotrichum lindemthianum);* leaf spot (*Cercospora personata*), leaf spot *(Cercospora arachidicola)* and southern blight *(Sclerotium rolfsii)* of peanut;
pea powdery mildew *(Erysiphe pisi);*
early blight *(Alternaria solani),* late blight (*Phytophthora infestans*) and *Verticillium* wilt *(Verticillium albo-atrum, V. dahliae, V. nigrescens)* of potato;
strawberry powdery mildew *(Sphaerotheca humuli);*
net blister blight *(Exobasidium reticulatum)*; white scab *(Elsinoe leucospila),* zonate leaf spot (*Pestalotiopsis* sp.) and anthracnose *(Colletotrichum theae-sinensis)* of tea plant;
brown spot *(Alternaria longipes),* powdery mildew (*Erysiphe cichoracearum*)*,* anthracnose (*Colletotrichum tabacum*), downy mildew *(Peronospora tabacina)* and *Phytophthora* rot *(Phytophthora nicotiana)* of tobacco;
leaf spot *(Cercospora beticola),* foliage blight *(Thanatephorus cucumeris)* and root rot *(Thanatephorus cucumeris)* of beet;
black spot *(Diplocarpon rosae)* and powdery mildew *(Sphaerotheca pannosa)* of rose;
leaf blight *(Septoria chrysanthemi-indici)* and white rust *(Puccinia horiana)* of chrysanthemum;
*Botrytis* diseases *(Botrytis cinerea, B. byssoidea, B. squamosa),* gray mold neck rot *(Botrytis alli)* and Small sclerotial neck rot *(Botrytis squamosa)* of onion;
gray mold *(Botrytis cinerea)* and stem rot *(Sclerotinia sclerotiorum)* of various crops;
*Alternaria* leaf spot *(Alternaria brassicicola)* of Japanese radish;
dollar spot *(Sclerotinia homeocarpa),* brown patch and large patch *(Rhizoctonia solani)* of turf grass; and Sigatoka diseases *(Mycosphaerella fijiensis, Mycosphaerella musicola, Pseudocercospora musae)* of banana.

### Examples

The present invention will be explained in more detail by way of Preparation Examples, Formulation Examples and Test Examples, which should not be construed as limiting the present invention. All the "parts" are by weight.

### Preparation Example 1 of the present compound

To 5 mL of pyridine were added 1.5 g of 2-aminothiazole-5-carboxylic acid hydrochloride, 0.49 g of 1-hydroxybenzotriazole, 0.69 g of WSC and 0.30 g of cyclohexylmethylamine, and the mixture was heated under reflux for 5 minutes, followed by stirring at room temperature for 4 hours. The reaction mixture was added to an aqueous saturated sodium bicarbonate solution, and extracted with ethyl acetate. The organic layer was washed successively with water and saturated brine, then dried over magnesium sulfate, and concentrated under reduced pressure. The resultant residue was subjected to silica gel column chromatography to obtain 0.55 g of N-cyclohexylmethyl-2-aminothiazole-5-carboxamide (hereinafter referred to as "the present compound (1)"). The present compound (1) 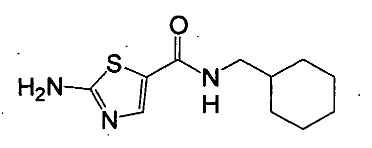 ¹H-NMR (CDCl₃) δ: 0.92-1.29 (5H, m), 1.52-1.76 (6H, m), 3.23 (2H, t, J = 6.2 Hz), 5.29 (2H, br s), 5.78 (1H, br s), 7.46 (1H, s).

### Preparation Example 2 of the present compound

To 4 mL of DMF were added 0.50 g of 2-amino-4-methylthiazole-5-carboxylic acid, 0.56 g of 1-hydroxybenzotriazole, 0.80 g of WSC and 0.47 g of cyclohexylmethylamine, and the mixture was stirred at 100°C for 2 hours. The reaction mixture was allowed to stand and cooled to about room temperature, added to an aqueous saturated sodium bicarbonate solution, and extracted with ethyl acetate. The organic layer was washed successively with water and saturated brine, then dried over magnesium sulfate, and concentrated under reduced pressure. The resultant residue was subjected to silica gel column chromatography to obtain 0.58 g of N-cyclohexylmethyl-2-amino-4-methylthiazole-5-carboxamide (hereinafter referred to as "the present compound (2)").
The present compound (2) 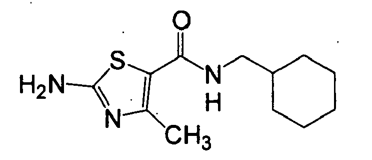 ¹H-NMR (CDCl₃) δ: 0.91-1.30 (5H, m), 1.49-1.76 (6H, m), 2.49 (3H, s), 3.22 (2H, t, J = 6.4 Hz), 5.28 (2H, br s), 5.54 (1H, br s).

### Preparation Example 3 of the present compound

To 15 mL of DMF were added 1.3 g of 2-aminothiazole-5-carboxylic acid hydrochloride, 0.50 g of 1-hydroxybenzotriazole,' 0.66 g of WSC, 0.32 g of (1S)-1-cyclohexylethylamine and 2.0 g of triethylamine, and the mixture was heated under reflux for 2 hours. The reaction mixture was allowed to stand and cooled to about room temperature, added to an aqueous saturated sodium bicarbonate solution, and extracted with ethyl acetate. The organic layer was washed successively with water and saturated brine, then dried over magnesium sulfate, and concentrated under reduced pressure. The resultant residue was subjected to silica gel column chromatography to obtain 0.26 g of N-[(1S)-1-cyclohexylethyl]-2-aminothiazole-5-carboxamide (hereinafter referred to as "the present compound (3)"). The enantiomeric excess of the resultant present compound (3) was 93%ee. The enantiomeric excess of the present compound (3) was measured using a chiral column (CHIRALCEL OF (4.6 mm in diameter x 25 cm in length) manufactured by Daicel Chemical Industries, Ltd.) under the conditions of column oven temperature: room temperature, UV wavelength of detector: 254 nm, flow rate: 1.0 mL/min, eluent: hexane/2-propanol = 2/1.

The present compound (3) 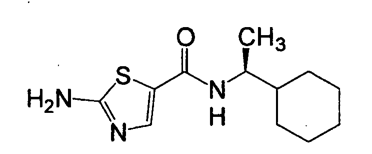 ¹H-NMR (CDCl₃) δ: 0.94-1.43 (8H, m), 1.65-1.79 (6H, m), 3.95-4.04 (1H, m), 5.19 (2H, br s), 5.49 (1H, d, J=8.2 Hz), 7.44 (1H, s).

### Preparation Example 4 of the present compound

To 10 mL of DMF were added 1.0 g of 2-[(1,1-dimethylethoxy)carbonylamino]-4-methylthiazole-5-carboxylic acid, 0.57 g of (1S)-1-cyclohexylethylamine and 1.0 g of WSC, and the mixture was stirred at room temperature for 8 hours. After addition of silica gel, the reaction mixture was concentrated under reduced pressure. The resultant residue was subjected to silica gel column chromatography to obtain 1.25 g of N-[(1S)-1-cyclohexylethyl]-2-[(1,1-dimethylethoxy)carbonylamino]-4-methylthiazole-5-carboxamide.

N-[(1S)-1-cyclohexylethyl]-2-[(1,1-dimethylethoxy)carbonylamino]-4-methylthiazole-5-carboxamide 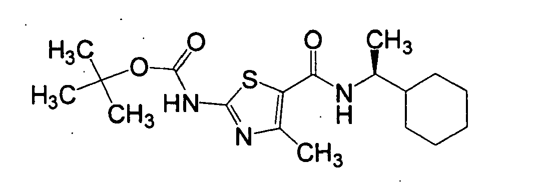 ¹H-NMR (CDCl₃) δ: 0.95-1.42 (8H, m), 1.49-1.83 (15H, m), 2.64 (3H, s), 3.96-4.05 (1H, m), 5.43 (1H, d, J=8.8 Hz), 10.37 (1H, br s).

To 5 mL of trifluoroacetic acid was added 0.96 g of N-[(1S)-1-cyclohexylethyl]-2-[(1,1-dimethylethoxy)carbonylamino]-4-methylthiazole-5-carboxamide, and the mixture was stirred at room temperature for 2 hours. The reaction solution was concentrated under reduced pressure. An aqueous saturated sodium bicarbonate solution was added to the resultant residue, followed by extraction with ethyl acetate. The organic layer was dried over magnesium sulfate, and concentrated under reduced pressure. The resultant residue was washed with hexane, followed by MTBE to obtain 0.57 g of N-[(1S)-1-cyclohexylethyl]-2-aminothiazole-4-methyl-5-carboxamide (hereinafter referred to as "the present compound (4)"). The enantiomeric excess of the resultant present compound (4) was 90%ee. The enantiomeric excess of the present compound (4) was measured using a chiral column (CHIRALCEL OF (4.6 mm in diameter x 25 cm in length) manufactured by Daicel Chemical Industries, Ltd.) under the conditions of column oven temperature: room temperature, UV wavelength of detector: 254 nm, flow rate: 1.0 mL/min, eluent: hexane/2-propanol = 2/1.

The present compound (4) 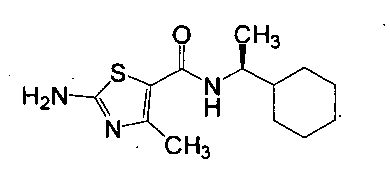 ¹H-NMR (CDCl₃) δ: 0.93-1.44 (8H, m), 1.63-1.81 (6H, m), 2.49 (3H, s), 3.94-4.03 (1H, m), 5.30 (1H, d, J = 8.8 Hz), 5.35 (2H, br s).

### Preparation Example 5 of the present compound

To a mixture of 0.30 g of 2-aminothiazole-5-carboxylic acid, 2 mL of DMF, 1.2 g of triethylamine and 0.44 g of cyclopropylmethylamine hydrochloride was added 1.1 g of BOP reagent, and the mixture was stirred overnight at room temperature. After addition of silica gel, the reaction mixture was concentrated under reduced pressure. The resultant residue was subjected to silica gel column chromatography. The resultant solid was washed with hexane, MTBE, an aqueous saturated sodium bicarbonate solution and water successively to obtain 0.085 g of N-cyclopropylmethyl-2-aminothiazole-5-carboxamide (hereinafter referred to as "the present compound (5)").

The present compound (5) 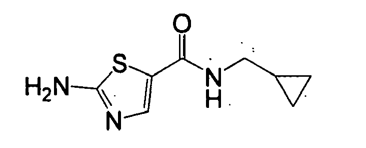 ¹H-NMR (DMSO-d₆) δ: 0.16-0.20 (2H, m), 0.39-0.43 (2H, m), 0.91-1.00 (1H, m), 3.03 (2H, dd, J=6.2, 6.2 Hz), 7.41 (2H, br s), 7.60 (1H, s), 8.15 (1H, t, J = 5.6 Hz).

### Preparation Example 6 of the present compound

To 10 mL of THF were added 0.80 g of 2-[(1,1-dimethylethoxy)carbonylamino]thiazole-5-carboxylic acid chloride, 0.33 g of 1-cyclobutylethylamine hydrochloride and 0.80 g of triethylamine, and the mixture was stirred at room temperature for 8 hours. Diluted hydrochloric acid was added to the reaction mixture, followed by extraction with ethyl acetate. The organic layer was washed with water and brine successively, dried over magnesium sulfate, and then concentrated under reduced pressure. The resultant residue was subjected to silica gel column chromatography to obtain 0.75 g of N-(1-cyclobutylethyl)-2-[(1,1-dimethylethoxy)carbonylamino]thiazole-5-carboxamide. N-(1-cyclobutylethyl)-2-[(1,1-dimethylethoxy)carbonylamino]thiazole-5-carboxamide 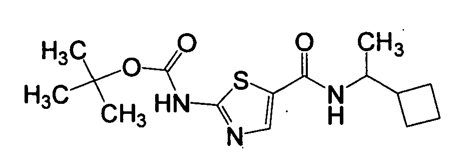 ¹H-NMR (CDCl₃) δ: 1.11 (3H, d, J = 6.3 Hz), 1.58 (9H, s), 1.74-2.07 (6H, m), 2.24-2.35 (1H, m), 4.07-4.16 (1H, m), 5.38 (1H, d, J = 8.5 Hz), 7.83 (1H, s), 10.63 (1H, br s).

To 5 mL of trifluroacetic acid was added 0.60 g of N-[(1-cyclobutyl)ethyl]-2-[(1,1-dimethylethoxy)carbonylamino]thiazole-5-carboxamide, and the mixture was stirred at room temperature for 4 hours. The reaction mixture was concentrated under reduced pressure, an aqueous saturated sodium bicarbonate solution was added to the resultant residue, and the solid formed was collected by filtration. The resultant solid was washed with MTBE to obtain 0.42 g of N-(1-cyclobutylethyl)-2-aminothiazole-5-carboxamide (hereinafter referred to as "the present compound (6)").

The present compound (6) 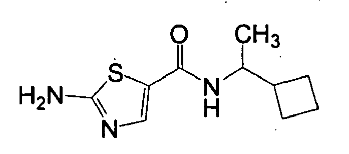 ¹H-NMR (DMSO-d₆) δ: 0.98 (3H, d, J = 6.6 Hz), 1.60-1.95 (6H, m), 2.26-2.37 (1H, m), 3.82-3.92 (1H, m), 7.38 (2H, br s), 7.61-7.62 (1H, m), 7.66-7.70 (1H, m).

### Preparation Example 7 of the present compound

To a mixture of 0.30 g of 2-aminothiazole-5-carboxylic acid, 2 mL of DMF, 1.2 g of triethylamine and 0.62 g of 1-cyclopentylethylamine hydrochloride was added 1.1 g of BOP reagent, and the mixture was stirred overnight at room temperature. An aqueous saturated sodium bicarbonate solution was added to the reaction mixture, followed by extraction with ethyl acetate. The organic layer was washed with water and saturated brine successively, then dried over magnesium sulfate, and concentrated under reduced pressure. The resultant solid was washed with ethyl acetate to obtain 0.12 g of N-(1-cyclopentylethyl)-2-aminothiazole-5-carboxamide (hereinafter referred to as "the present compound (7)").

The present compound (7) 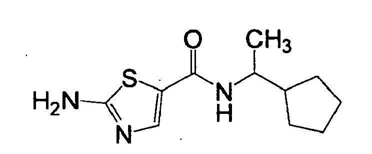 ¹H-NMR (DMSO-d₆) δ: 1.08 (3H, d, J = 6.5 Hz), 1.13-1.28 (2H, m), 1.43-1.70 (6H, m), 1.84-1.93 (1H, m), 3.69-3.78 (1H, m), 7.37 (2H, br s), 7.62 (1H, s), 7.78 (1H, d, J = 8.7 Hz).

### Preparation Example 8 of the present compound

To a mixture of 0.30 g of 2-amino-4-methylthiazole-5-carboxylic acid, 1.1 g of triethylamine and 0.64 g of 1-cyclobutylethylamine hydrochloride was added 1.0 g of BOP reagent, and the mixture was stirred overnight at room temperature. Water was added to the reaction mixture, followed by extraction with ethyl acetate. The organic layer was washed with an aqueous saturated sodium bicarbonate solution and saturated brine successively, then dried over magnesium sulfate, and concentrated under reduced pressure. The resultant residue was subjected to silica gel column chromatography to obtain 0.14 g of N-(1-cyclobutylethyl)-2-amino-4-methylthiazole-5-carboxamide (hereinafter referred to as "the present compound (8)"). The present compound (8) 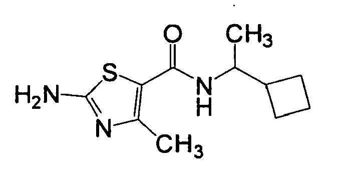 ¹H-NMR (DMSO-d₆) δ: 0.97 (3H, d, J = 6.6 Hz), 1.60-1.99 (6H, m), 2.28 (3H, s), 2.30-2.38 (1H, m), 3.81-3.90 (1H, m), 7.10 (1H, d, J = 8.5 Hz), 7.25 (2H, br s).

### Preparation Example 9 of the present compound

To a mixture of 0.30 g of 2-amino-4-methylthiazole-5-carboxylic acid, 1.1 g of triethylamine and 0.56 g of 1-cyclopentylethylamine hydrochloride was added 1.0 g of BOP reagent, and the mixture was stirred overnight at room temperature. Water was added to the reaction mixture, followed by extraction with ethyl acetate. The organic layer was washed with an aqueous saturated sodium bicarbonate solution and saturated brine successively, then dried over magnesium sulfate, and concentrated under reduced pressure. The resultant solid was subjected to recrystallization with ethyl acetate to obtain 0.09 g of N-(1-cyclopentylethyl)-2-amino-4-methylthiazole-5-carboxamide (hereinafter referred to as "the present compound (9)"). The present compound (9) 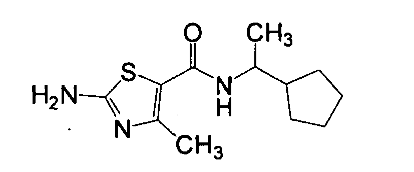 ¹H-NMR (DMSO-d₆) δ: 1.07 (3H, d, J = 6.5 Hz), 1.10-1.27 (2H, m), 1.42-1.70 (6H, m), 1.86-1.95 (1H, m), 2.29 (3H, s), 3.-67-3.75 (1H, m), 7.21 (1H, d, J = 8.7 Hz), 7.24 (2H, br s).

### Preparation Example 10 of the present compound

To a mixture of 0.30 g of 2-aminothiazole-5-carboxylic acid, 2 mL of DMF, 1.2 g of triethylamine and 0.56 g of cyclopentylmethylamine hydrochloride was added 1.1 g of BOP reagent, and the mixture was stirred overnight at room temperature. An aqueous saturated sodium bicarbonate solution was added to the reaction mixture, followed by extraction with ethyl acetate. The organic layer was washed with water and saturated brine successively, then dried over magnesium sulfate, and concentrated under reduced pressure. The resultant residue was subjected to silica gel column chromatography to obtain 0.12 g of N-cyclopentylmethyl-2-aminothiazole-5-carboxamide (hereinafter referred to as "the present compound (10)"). The present compound (10) 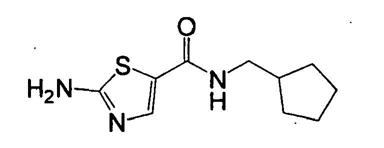 ¹H-NMR (DMSO-d₆) δ: 1.15-1.25 (2H, m), 1.44-1.69 (6H, m), 2.03-2.10 (1H, m), 3.07 (2H, dd, J = 7.2, 6.0 Hz), 7.39 (2H, br s), 7.59 (1H, s), 8.05 (1H, t, J = 5.6 Hz).

### Preparation Example 11 of the present compound

To a mixture of 0.30 g of 2-amino-4-methylthiazole-5-carboxylic acid, 2 mL of DMF, 1.1 g of triethylamine and 0.51 g of cyclopentylmethylamine hydrochloride was added 1.0 g of BOP reagent, and the mixture was stirred overnight at room temperature. An aqueous saturated sodium bicarbonate solution was added to the reaction mixture, followed by extraction with ethyl acetate. The organic layer was washed with water and saturated brine successively, then dried over magnesium sulfate, and concentrated under reduced pressure. The resultant residue was subjected to silica gel column chromatography to obtain 0.11 g of N-cyclopentylmethyl-2-amino-4-methylthiazole-5-carboxamide (hereinafter referred to as "the present compound (11)").

The present compound (11) 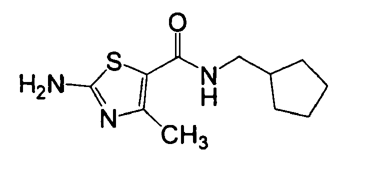 ¹H-NMR (DMSO-d₆) δ: 1.16-1.26 (2H, m), 1.45-1.67 (6H, m), 2.04-2.12 (1H, m), 2.30 (3H, s), 3.02-3.09 (2H, m), 7.27 (2H, br s), 7.46 (1H, t, J = 5.0 Hz).

### Preparation Example 1 of intermediate

To 10 mL of THF were added 0.8 g of 2-[(1,1-dimethylethoxy)carbonylaminol thiazole-5-carboxylic acid chloride, 0.27 g of (1S)-1-cyclohexylethylamine and 0.80 g of triethylamine, and the mixture was stirred at room temperature for 8 hours. Water was added to the reaction mixture, followed by extraction with ethyl acetate. The organic layer was washed with an aqueous saturated sodium bicarbonate and saturated brine successively, then dried over magnesium sulfate, and concentrated under reduced pressure. The resultant solid was washed with hexane to obtain 0.94 g of N-[(1S)-1-cyclohexylethyl]-2-[(1,1-dimethylethoxy)carbonylamino]thiazole-5-carboxamide.

### N-[(1S)-1-cyclohexylethyl]-2-[(1,1-dimethylethoxy)carbonylamino]thiazole-5-carboxamide

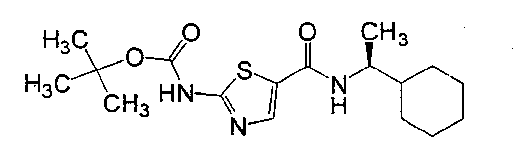

¹H-NMR (CDCl₃) δ: 0.97-1.44 (8H, m), 1.48-1.81 (15H, m), 4.00-4.05 (1H, m), 5.48 (1H, d, J = 8.9 Hz), 7.84 (1H, s), 10.49 (1H, br s).

### Formulation Example 1

Fifty parts of any one of the present compounds (1)-(11), 3 parts of calcium ligninsulfonate, 2 parts of magnesium laurylsulfate, and 45 parts of synthetic hydrous silicon oxide are thoroughly ground and mixed to obtain a wettable powder.

### Formulation Example 2

Twenty parts of any one of the present compounds (1)-(11) and 1.5 parts of sorbitan trioleate are mixed with 28.5 parts of an aqueous solution containing 2 parts of polyvinyl alcohol, and the mixture is pulverized by wet pulverizing method. Then 40 parts of an aqueous solution containing 0.05 parts of xanthan gum and 0.1 parts of aluminum magnesium silicate are added thereto, and further added 10 parts of propylene glycol, followed by stirring and mixing to obtain a flowable formulation.

### Formulation Example 3

Two parts of any one of the present compounds (1)-(11), 88 parts of kaolin clay and 10 parts of talc are thoroughly ground and mixed to obtain a dust formulation.

### Formulation Example 4

Five parts of any one of the present compounds (1)-(11), 14 parts of polyoxyethylenestyrylphenyl ether, 6 parts of calcium dodecylbenzenesulfonate, and 75 parts of xylene are thoroughly mixed to obtain an emulsifiable concentrate.

### Formulation Example 5

Two parts of any one of the present compound (1)-(11), 1 part of synthetic hydrous silicon oxide, 2-parts of calcium ligninsulfonate, 30 parts of bentonite and 65 parts of kaolin clay are thoroughly ground and mixed, then water is added thereto, followed by thoroughly kneading and granulation drying to obtain a granule formulation.

### Formulation Example 6

Ten parts of any one of the present compound (1)-(11), 35 parts of white carbon containing 50 parts of polyoxyethylene alkyl ether sulfate ammonium salt, and 55 parts of water are mixed, and the mixture is pulverized by wet pulverizing method to obtain a dust formulation.

### Formulation Example 7

Forty parts of any one of the present compounds (1)-(11), 5 parts of propylene glycol (manufactured by Nakarai Tesque Inc.), 5 parts of Soprophor FLK (manufactured by Rhodia Nikka Co., Ltd.), 0.2 parts of Antifoam C Emulsion (manufactured by Dow Corning Corporation), 0.3 parts of Proxel GXL (manufactured by Arch Chemicals Inc.) and 49.5 parts of ion exchange water are mixed to prepare a bulk slurry. To 100 parts of the slurry is added 150 parts of glass beads (1 mm in diameter), followed by pulverization for 2 hours under cooling with cooling water. After pulverization, the glass beads are removed by filtration to obtain a flowable formulation for seed treatment.

After mixing, water is added thereto, followed by thoroughly kneading and granulation drying to obtain a granule formulation.

### Formulation Example 8

Fifty parts of any one of the present compounds (1)-(11), 38.5 parts of NN kaolin clay (manufactured by Takehara Kagau Kogyo Co., Ltd.), 10 parts of Morwet D425 and 1.5 parts of Morwer EFW (manufactured by Akzo Nobel) are mixed to obtain AI premix. The premix is ground with a jet mill to obtain a dust formulation for dry seed treatment.

Hereinafter, usefulness of the present compounds for controlling plant diseases is shown by test examples.

Here, the controlling effect was evaluated by comparing the area of lesions on test plants treated with the present compound with that on untreated plants through visual observation of the area of lesion on the test plant at testing.

### Test Example 1

### Test on prevention effect against late blight of tomato (Phytophthora infestans)

Sand soil was packed in a plastic pot, and seeds of tomato (variety: Patio) were sown therein and grown for 20 days in a greenhouse. The seedlings were transplanted in another plastic pot, and grown for another 15 days in the greenhouse. Any one of the present compounds (1)-(4) and (6)-(10) was processed into a flowable formulation in accordance with Formulation Example 6. The formulation was then diluted with water to form a solution with a specified concentration (500 ppm), and the solution was sprayed over the foliar part of the tomato seedlings so that a sufficient amount of the solution would be applied to the surface of leaves of the tomato seedling. After air-drying to an extent that the diluted solution on the leaves was dried, an aqueous suspension of the spores of *Phytophthora infestans* was spray-inoculated. After the inoculation, the seedlings were placed at 12°C for 1 day under high humidity, and further incubated for 4 days in the greenhouse. Then, the area of lesions was examined.

The lesion areas on the plant treated with the present compounds (1)-(4) and (6)-(10) were less than 30% of the lesion area on the untreated plant.

### Industrial Applicability

As described hereinabove, the present compound has excellent plant disease controlling effect and hence is useful as an active ingredient of plant disease controlling agents.

## Claims

1. An amide compound represented by the formula (1): 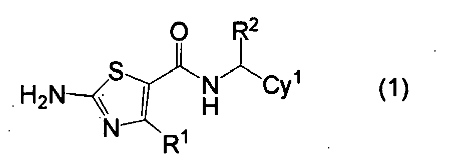 wherein R¹ and R² independently represent a hydrogen atom or a methyl group; and Cy¹ represents a C3-C6 cycloalkyl group.

2. The amide compound according to claim 1, wherein Cy¹ represents a cyclohexyl group.

3. The amide compound according to claim 1 or 2, wherein R¹ represents a hydrogen atom.

4. The amide compound according to any one of claims 1 to 3, wherein R² represents a hydrogen atom.

5. The amide compound according to claim 1, wherein R¹ represents a hydrogen atom, R² represents a methyl group, and Cy¹ represents a cyclohexyl group.

6. A plant disease controlling agent, which comprises the amide compound according to any one of claims 1 to 5 as an active ingredient.

7. A method for controlling plant diseases, which comprises the step of applying an effective amount of the amide compound according to any one of claims 1 to 5 to plants or soils.

8. Use of the amide compound according to any one of claims 1 to 5 for controlling plant diseases by means of applying the amide compound to plants or soils.

## Patentansprüche

1. Eine Amidverbindung, dargestellt durch, die Formel (1): 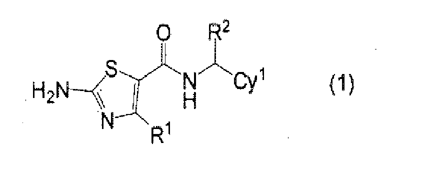 wobei R₁ und R₂ unabhängig ein Wasserstoffatom oder eine Methylgruppe darstellen, und Cy¹ einen C3-C6 Cycloalkylrest darstellt.

2. Die Amidverbindung gemäß Anspruch 1, wobei Cy¹ eine Cyclohexylgruppe darstellt.

3. Die Amidverbindung gemäß Anspruch 1 oder 2, wobei R¹ ein Wasserstoffatom darstellt.

4. Die Amidverbindung gemäß einem der Ansprüche 1 bis 3, wobei R² ein Wasserstoffatom darstellt.

5. Die Amidverbindung gemäß Anspruch 1, wobei R¹ ein Wasserstoffatom darstellt, R² eine Methylgruppe darstellt, und Cy¹ eine Cyclohexylgruppe darstellt.

6. Ein Mittel zur Bekämpfung von Pflanzenkrankheiten, welches die Amidverbindung gemäß einem der Ansprüche 1 bis 5 als Wirkstoff umfasst.

7. Ein Verfahren zur Bekämpfung von Pflanzenkrankheiten, welches den Schritt des Aufbringens einer wirksamen Menge der Amidverbindung gemäß einem der Ansprüche 1 bis 5 auf Pflanzen oder Böden umfässt.

8. Verwendung der Amidverbindung gemäß einem der Ansprüche 1 bis 5 zur Bekämpfung von Pflanzenkrankheiten durch Aufbringen der Amidverbindung auf Pflanzen oder Böden.

## Revendications

1. Composé amide représenté par la formule (1) : 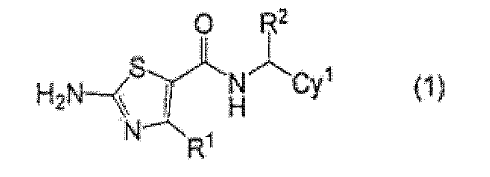 où R¹ et R² représentent indépendamment un atome d'hydrogène ou un groupe méthyle ; et Cy¹ représente un groupe C3-C6 cycloalkyle.

2. Composé amide selon la revendication 1 où Cy¹ représente un groupe cyclohexyle.

3. Composé amide selon la revendication 1 ou 2 où R¹ représente un atome d'hydrogène.

4. Composé amide selon l'une quelconque des revendications 1 à 3 où R² représente un atome d'hydrogène.

5. Composé amide selon la revendication 1 où R¹ représente un atome d'hydrogène, R² représente un groupe méthyle et Cy¹ représente un groupe cyclohexyle.

6. Agent de lutte contre les maladies des plantes qui comprend le composé amide selon l'une quelconque des revendications 1 à 5 comme ingrédient actif.

7. Procédé pour lutter contre les maladies des plantes qui comprend l'étape d'application d'une quantité efficace du composé amide selon l'une quelconque des revendications 1 à 5 à des plantes ou à des sols.

8. Utilisation du composé amide selon l'une quelconque des revendications 1 à 5 pour lutter contre les maladies des plantes par application du composé amide à des plantes ou à des sols.
